Europäisches Patentamt

(19) European Patent Office

Office européen des brevets

(11) Publication number: **0 339 193 B1**

(12) **EUROPEAN PATENT SPECIFICATION**

(45) Date of publication of patent specification :
29.01.92 Bulletin 92/05

(51) Int. Cl.$^5$ : **C07K 7/06, A61K 37/02**

(21) Application number : **89102283.2**

(22) Date of filing : **10.02.89**

(54) Peptide ligands for bombesin receptors.

The file contains technical information
submitted after the application was filed and
not included in this specification

(30) Priority : **14.04.88 GB 8808768**

(43) Date of publication of application :
**02.11.89 Bulletin 89/44**

(45) Publication of the grant of the patent :
**29.01.92 Bulletin 92/05**

(84) Designated Contracting States :
**DE GB IT**

(56) References cited :
WO-A-89/02897
FR-A- 2 100 975
US-A- 4 207 311
US-A- 4 331 661
US-A- 4 613 586
CHEMICAL ABSTRACTS, vol. 107, 1987, page
100, abstract no. 90190s, Columbus, Ohio, US;
D.A. WALLACE et al.: "Chemotactic activity of
peptide fragments of bombesin and gastrin",
& BIOCHEM. SOC. TRANS. 1987, 15(5), 928
UNLISTED DRUGS, vol. 31, no. 11, November
1979, page 161-h, no. B9, Philadelphia, US;
"Biologically active bombesin fragment used
to obtain antibodies (from B 9-immunized rab-
bits) for radioimmune assay of mammalian
bombesin-like peptides"

(56) References cited :
EXPERIENTIA, vol. 31, no. 5, 1975, pages
507-508; F. ANGELUCCI et al.: "H2S as sulfur
source in Lemna minor L.: II. Direct incorpor-
ation into cysteine and inhibition of sulfate
assimilation"
Heinz-Erian, P. et al. (1987) Am. J. of Physiol.,
252, pg. G439-G442

(73) Proprietor : **FARMITALIA CARLO ERBA S.r.L.**
**Via Carlo Imbonati 24**
**I-20159 Milano (IT)**

(72) Inventor : **de Castiglione, Roberto**
**Via Domenichino, 38**
**I-20100 Milan (IT)**
Inventor : **Gozzini, Luigia**
**Via degli Anemoni, 19**
**I-20100 Milan (IT)**
Inventor : **Mena, Renzo**
**Via dei Caduti, 1**
**I-28066 Galliate (Novara) (IT)**
Inventor : **Brugnolotti, Manuela**
**Via Marco d'Agrate, 21**
**I-26100 Cremona (IT)**
Inventor : **Ciomei, Marina**
**Via Riviera**
**I-27020 Massaua-Torre d'Isola (Pavia) (IT)**
Inventor : **Molinari, Isabella**
**Via Elba, 28**
**I-20100 Milan (IT)**

(74) Representative : **Ferrario, Vittorino**
**c/o Farmitalia Carlo Erba S.r.l. Via Carlo**
**Imbonati 24**
**I-20159 Milano (IT)**

EP 0 339 193 B1

**Description**

The invention relates to biologically active peptides and their pharmaceutically acceptable salts. to processes for their preparation and to pharmaceutical compositions containing them. Experientia, vol 31/5, 1975, pages 507-508 deals with the synthesys of litorin hydrochloride by deprotection of the intermediate of the formula H-Glp-Gln-Trp-Ala-Val-Gly-His(Dnp)-Phe-Met-NH$_2$. US-A-4207311 relates to peptides useful as analgesic and for thermoregulation control of the formula R$_1$-R$_2$-Trp--Ala-Val-R$_3$-His-R$_4$-R$_5$-NH$_2$ wherein R, may be hydrogen, an amino acid, or a peptide with 2 to 6 amino acids including Glp ; R$_2$ is D-Glp, D-Gln and Gln ; R$_3$ is D-Ala or Gly, R$_4$ is Phe or Leu and R$_5$ is Met or D-Met.

In this Specification symbols and abbreviations are those commonly used in peptide chemistry (see Eur. J. Biochem, (1981), 138, 9-37), Consequently the three-letter amino acid symbols denote the L configuration of chiral amino acids. D-amino acids are represented by the presence of D before the symbol : e.g. D-Ala. Other symbols and abbreviations used are Bzl benzyl ; BBS. bombesin. CCD, counter-currrent distribution ; DCC. N.N′-dicyclohexylcarbodiimide : DCEU. N,N′-dicyclohexylurea ; dec, decomposition : DMAP. 4-dimethylaminopyridine ; DMF. dimethylformamide ; Dnp. 2,4-dinitrophenyl ; ECC, ethylchlorocarbonate Glp. L-pyroglutamic acid. h-GRP (or p-GRP), human (or porcine) gastrin releasing peptide : HCl/AcOH, dry HCl in anhydrous acetic acid ; HOBt, 1-hydroxybenzotriazole ; i.c.v., intracerebroventricular(ly) ; Me, methyl ; m.p., melting point ; n.d., not determined ; NMM, N-methylmorpholine ; OSu. N-hydroxysuccinimidyl : TLC, thin layer chromatography : Tos, p-toluenesulphonyl : Z, benzyloxycarbonyl.

The invention provides peptides of the general formula I A-B-Gln-Trp-Ala-Val-W-X-Y-T wherein
A represents a hydrogen atom, a Boc group or a Lys or Arg residue,
B represents a valence bond or an Asn, Thr or Glp residue,
W represents a Gly or ala residue,
X represents a valence bond or a His (R$_1$), his (R$_1$), Phe, Ser, D-Ser, Ala or D-Ala residue.
Y represents a valence bond or a Leu, D-Leu, Phe or D-Phe residue,
T represents an NH$_2$, NH(CH$_2$)$_4$CH$_3$ or NHCH$_2$ C$_6$H$_5$ group or a Met-R$_2$, Leu-R$_2$, Ile-R$_2$ or Nle-R$_2$ residue,
R$_1$ represents a hydrogen atom or a Tos, Drp or Bzl group, and
R$_2$ represents an NH$_2$, OH, OMe or NH-NH$_2$ group.
with the provisos that
when A represents a Lys or Arg residue, then B does not represent a Glp residue,
when X represents a His residue, then Y-T together do not represent Leu-Met-NH$_2$ or Phe-Met-NH$_2$ residues,
when X represents a His (Dnp) residue and A represents a βoc group, then β does not represent an Asn residue, and
when X represents a His (Dnp) residue, then β does not represent a Glp residue.

Salts of peptides according to the invention with pharmaceutically acceptable acids are within the scope of the invention. Such acid addition salts can be derived from a variety of inorganic and organic acids such as sulphuric, phosphoric, hydrochloric, hydrobromic, hydroiodic, nitric, sulphamic, citric, lactic, pyruvic, oxalic, maleic, succinic, tartaric, cinnamic, acetic, trifluoracetic, benzoic, salicylic, gluconic, ascorbic and related acids.

The synthesis of the peptides of the invention may be accomplished by classical solution methods. The synthesis consists essentially of appropriate successive condensations of protected amino acids or peptides. The condensations are carried out so that the resulting peptides have the desired sequence of amino acid residues.

The amino acids and peptides, which can be condensed according to methods known in polypeptide chemistry, have their amino and carboxyl groups, which are not involved in the formation of the peptide linkage, blocked by protecting groups capable of being removed by acid or alkali treatment or by hydrogenolysis.

For the protection of the amino group the following protective groups may, for example, be employed : benzyloxycarbonyl, t-butoxycarbonyl, trityl, formyl, trifluoracetyl, o-nitrophenylsulphenyl, 4-methyloxybenzyloxycarbonyl, 9-fluorenylmethoxycarbonyl, 3,5-dimethoxy-α,α′-dimethylbenzyloxycarbonyl or methylsulphonylethoxycarbonyl.

For the protection of the carboxyl group the following protective groups may, for example, be employed : methyl, ethyl, t-butyl, benzyl, p-nitrobenzyl, fluorenylmethyl, amide, hydrazide, t-butoxycarbonyl hydrazide or benzyloxycarbonyl hydrazide.

The hydroxy functions of hydroxy amino acids and the imino function of histidine may be protected by suitable protecting groups (throughout all the synthesis or only during a few steps) or may be unprotected.

For the protection of the hydroxy function the following protective groups may, for example, be employed; t-butyl, benzyl, acetyl. For the protection of the hydroxy function the following groups may, for example, be used; 2,4-dinitrophenyl, tosyl, benzyl.

2

De-protecting reactions are carried out according to methods known per se in polypeptide chemistry.

The condensation between an amino group of one molecule and a carboxyl group of another molecule to form the peptidic linkage may be carried out through an activated acyl-derivative such as a mixed anhydride, an azide or an activated ester, or by a direct condensation between a free amino group and a free carboxyl group, in the presence of a condensing agent such as dicyclohexylcarbodiimide, alone or together with a racemization preventing agent, such as N-hydroxysuccinimide or 1-hydroxybenzotriazole, or together with an activating agent such as 4-dimethylamino-pyridine. The condensation may be carried out in a solvent such as dimethylformamide, dimethylacetamide, pyridine, acetonitrile, tetrahydrofuran or N-methyl-2-pyrrolidone. The reaction temperature may be from $-30°C$ to ambient temperature. The reaction time is generally from 1 to 120 hours. The scheme of synthesis, the protecting groups and condensing agents are selected so as to avoid the risk of racemization.

The compounds according to the invention are ligands for bombesin receptors.

Bombesin (BBS) is a tetradecapeptide of formula Glp-Gln-Arg-Leu-Gly-Asn-Gln-Trp-Ala-Val-Gly-His-Leu-Met-$NH_2$ originally isolated from the skin of a frog. The biological activity resides in the C-terminal part of the molecule. BBS (6-14) nonapeptide is as active as the parent compound. The human counterpart of bombesin is a 27 amino acid peptide known as gastrin-releasing peptide (h-GRP). Bombesin and bombesin-like peptides display a number of biological activities (J.H. Walsh (1983) in 'Brain Peptides, D.T. Krieger, M.J. Brownstein and J.B. Martin (eds.), Wiley Interscience Publ, pp. 941-960), including autocrine growth-promoting effects on human small cell lung carcinoma (SCLC) (F. Cuttitta et al, (1985), Cancer Survey, 4, 707-727).

Quite surprisingly compounds of the present invention are competitive antagonists for bombesin receptors. They can, therefore, conceivably find therapeutical applications in the treatment of human SCLC and/or in the management of the clinical symptoms associated with this disease and due to hypersecretion of this peptide hormone.

The binding affinity of the compounds of the present invention for the bombesin receptors has been determined on Swiss 3T3 fibroblasts (I. Zachary and E. Rozengurt (1985) Proc. Natl. Acad. Sci. USA, 82, 7616-7620) (Table 1).

The antagonistic activity has been evaluated both in vitro and in vivo ; in vitro, as inhibition of the bombesin-induced DNA synthesis in Swiss 3T3 cells (A.N. Corps et al. (1985) Biochem. J., 231, 781-785) (Table 2) and rat urinary bladder contraction (M. Broccardo et al. (1975) Br. J.Pharmac., 55, 221-227) (Table 3) ; in vivo, by i.c.v. administration, as inhibition of the rat grooming behaviour stimulated by bombesin (A. Cowan et al. (1985) Life Sciences, 37, 135-145) (Table 4).

Melting points are determined in open capillaries with a Tottoli apparatus and are uncorrected. Most of the derivatives soften and decompose before melting. Solvents for crystallization, precipitation or grinding are reported in brackets.

$R_f$ values are determined on pre-coated plates of silica gel 60 $F_{254}$ (Merck), layer thickness 0.25 mm, length 20 cm, using the following development systems :

System A : ethyl acetate/benzene/acetic acid/water = 500/500/100/50 by volume (upper phase)
System B : ethyl acetate/benzene/acetic acid/water = 500/500/200/75 by volume (upper phase)
System C : n-butanol/acetic acid/water = 600/150/150 by volume
System D : chloroform/methanol/$NH_4OH$ 30% = 448/388/150 by volume
System E : chloroform/methanol = 450/50 by volume
System F : benzene/petroleum ether 60°-80°/ethyl acetate = 250/50/700 by volume

TLC analyses are carried out at a temperature ranging from 18 to 25°C : the $R_f$ values can therefore change $\pm$ 5%.

High voltage paper electrophoresis is carried out with a Pherograph-Original-Frankfurt Type 64 apparatus on Schleicher and Schüll paper No 2317 at pH 1.2 (formic acid : acetic acid : water = 123 : 100 : 777) at 1600 V (40V/cm). The products are characterized by their mobilities relative to Glu.

High performance liquid chromatography (HPLC) was carried out using a Hewlett-Packard 1084B apparatus equipped with a UV detector operating at 210 nm.

The peptides are separated on a 4 × 250 mm Lichrosorb RP 18 5 µ column. The following solvents are used :

A) 0.02 M $KH_2PO_4$ adjusted at pH 3.5 with 3% $H_3PO_4$/$CH_3CN$ 9/1 by volume.
B) 0.02 M $KH_2PO_4$ adjusted to pH 3.5 with 3% $H_3PO_4$/$CH_3CN$ 3/7 by volume.

The elution is programmed with a linear gradient from 10% B to 90% B over a period of 20 min, and then isocratically for 15 min, with a flow rate of 1 ml/min. The peptides are characterized by their retention time (RT).

## TABLE 1

Binding affinity of Bombesin analogs on mouse Swiss 3T3 fibroblasts.

| COMPOUND | $ID_{50}$ (nM) |
|---|---|
| XVIII | 1,000 ± 100 |
| XIX | 3,400 |
| XX | 63,000 |
| XXI | 10,000 ± 3,900 |
| XXII | 5,800 ± 750 |
| XXIII | 4,700 ± 1,000 |
| XXIV | 590 ± 130 |
| XXV | 970 ± 4 |
| XXVI | 200 ± 60 |
| XXVII | 335 ± 101 |
| XXVIII | 76 ± 30 |
| XXXII | 770 ± 13 |
| XXXIII | 556 ± 128 |
| XXXIV | 18,200 ± 7,300 |
| BBS | 12.6 ± 3.8 |
| BBS(6-14)nonapeptide | 9.4 ± 3.1 |
| p-GRP | 17.6 ± 4.0 |

4

## TABLE 2

### [H³]Thymidine incorporation in mouse Swiss 3T3 fibroblasts

| Molarity Compound | Fold increase over basal values | | | | % inhibition | | | |
|---|---|---|---|---|---|---|---|---|
| | | | | | in the presence of 5 nM BBS | | in the presence of 25 nM BBS | |
| | 5 nM | 50 nM | 500 nM | 5000 nM | 500 nM | 5000 nM | 500 nM | 5000 nM |
| XVIII | 1 | 1 | 1.2 | 1.1 | 0 | 43 ± 10 | 18 | 53 ± 14 |
| XIX | 1 | 1 | 1.7 | 1.2 | 29 ± 13 | 10 ± 2 | 39 ± 20 | 32 ± 9 |
| XXVII | nd | 1 | 1 | 1 | nd | nd | nd | nd |
| XXXII | nd | 1 | 0.8 | 1.1 | nd | nd | 9.8 | 18.1 |
| XXXIII | nd | 1.4 | 1.8 | 1.5 | nd | nd | 0 | 15.5 |
| | 5 nM | 25 nM | | | | | | |
| BBS | 3.0±1 | 6.6±2.6 | | | | | | |
| p-GRF | 3.3±0.7 | 5.8±0.7 | | | | | | |

TABLE 3

Rat urinary bladder: % contraction as compared to 0.04 μM BBS = 100 (A); % inhibition of the contraction induced by 0.04 μM BBS (B).

| Observed effect | A | | | B | | |
|---|---|---|---|---|---|---|
| Compound ⟍ Molarity | 0.5 μM | 1 μM | 5 μM | 0.5 μM | 1 μM | 5 μM |
| XVIII | 0 | 0 | 19.0 | 0 | 0 | 0 |
| XIX | 0 | 0 | 20.9 | 0 | 12.5 | 0 |

TABLE 4

Grooming behaviour in the rat by i.c.v. administration of BBS (0.01 μg/rat) and peptide XXI (10 μg/rat), alone and in combination.

| Compound | Dose μg | Grooming score |
|---|---|---|
| XXI | 10 | 15 |
| BBS | 0.01 | 55 |
| BBS + XXI | 0.01 10 | 35 |

The following examples are given to illustrate the invention without limiting it.

## EXAMPLE 1

Preparation of Boc-Thr-Gln-Trp-Ala-Val-Gly-Leu-Met-Nh$_2$ (XVIII)

Step 1 Boc-Val-Gly-OBzl (I)

To a solution of 43.45 g (200 mmol) of Boc-Val-OH in 500 ml of anhydrous THF, 22.48 ml (200 mmol) of NMM and 19.80 ml (200 mmol) of ECC were successively added at a temperature of −15°C. After stirring at this temperature for 2 minutes, a cold solution of 67.47 g (200 mmol) of H-Gly-OBzl.Tos-OH and 22.48 ml (200 mmol) of NMM in 500 ml of anhydrous DMF was added.

The reaction mixture was stirred for 45 minutes at −12°C and for 60 minutes at 0°C, then filtered from salts and evaporated in vacuo. The residue was dissolved in ethyl acetate and washed several times successively with a 10% solution of citric acid, brine, a 5% solution of sodium bicarbonate and brine again.

The organic layer was dried over anhydrous sodium sulphate and the solvent removed in vacuo, obtaining a partially purified compound as an oil.

The oil was ground in petroleum ether, obtaining 60.50 g of pure solid compound I (83% yield) : m.p. 76-78°C ; $R_{fA}$ 0.81.

Step 2 HCl.H-Val-Gly-OBzl (II)

56.40 g of Boc-Val-Gly-OBzl (I) (154.75 mmol) were dissolved in 570 ml of 1.33 M HCl/AcOH with magnetic stirring at room temperature. After 40 the minutes Boc removal was completed and the solution was evaporated in vacuo, obtaining a crude product as an oil.

The product was evaporated in vacuo twice from anhydrous DMF. 44.2 g of oily compound II (95% yield) were obtained : $R_{fA}$ 0.62.

Step 3 Boc-Ala-Val-Gly-OBzl (III)

Boc-Ala-OH (27.81 g, 147 mmol) and HCl.H-Val-Gly-OBzl (II) (44.21 g, 147 mmol) were condensed as described in this example, step 1.

The crude oil was dissolved in CH$_2$Cl$_2$ and washed several times successively with a 10% solution of citric acid, brine, 5% sodium bicarbonate and brine again. The organic layer was dried over anhydrous sodium sulphate and the solvent removed in vacuo.

The residue was dissolved in a mixture of CH$_2$Cl$_2$ and methanol, and precipitated by addition of petroleum ether. 54.82 g (86% yield) of compound III was obtained : m.p. 142-146°C ; $R_{fE}$ 0.81.

Step 4 HCl.H-Ala-Val-Gly-OBzl (IV)

Boc-Ala-Val-Gly-OBzl (III) (27 g, 62 mmol) was deblocked as described in this example, step 2. The crude oil was dissolved in methanol and ethyl acetate and precipitated by addition of petroleum ether. 22.65 g (98% yield) of solid compound IV were obtained : m.p. 178-181°C ; $R_{fC}$ 0.47.

Step 5 Boc-Trp-Ala-Val-Gly-OBzl (V)

Boc-Trp-OH (16.48 g, 54.16 mmol) and HCl.H-Ala-Val-Gly-OBzl (IV) (20.14 g, 54.16 mmol) were condensed as described in this example, step 1. The crude oil, dissolved in DMF, was poured dropwise in a 10% solution of citric acid at 0°C. After filtration and washings with water to neutrality, the precipitate was redissolved in a mixture of CH$_2$Cl$_2$ and methanol, and precipitated by the addition of diethyl ether. 26.36 g (78% yield) of solid compound V were obtained : m.p. 154-177°C (dec.) ; $R_{fA}$ 0.73.

Step 6 HCl.H-Trp-Ala-Val-Gly-OBzl (VI)

20 g of Boc-Trp-Ala-Val-Gly-OBzl (V) (32.17 mmol) were dissolved in a mixture of 20 ml anisole, 10 ml of 2-mercaptoethanol and 200 ml of 1.33 M HCl/AcOH with magnetic stirring at room temperature.

After 30 minutes the Boc-deblocking was completed and the solvent was evaporated in vacuo, obtaining a solid residue that was dissolved in anhydrous DMF and evaporated 3 times consecutively.

The product was crushed with methanol, completing the precipitation with diethyl ether. 17.73 g (98% yield) of compound VI were obtained : m.p. 118-122°C ; $R_{fC}$ 0.73.

Step 7 Boc-Gln-Trp-Ala-Val-Gly-OBzl (VII)

11.73 g of Boc-Gln-OH (47.66 mmol) and 26.60 g of HCl. H-Trp-Ala-Val-Gly-OBzl (VI) (47.66 mmol) were condensed as described in this example, step 1. The crude oil was dissolved in DMF and poured dropwise in a cold solution of 10% citric acid.

The precipitate was filtered and washed with water to neutrality.

After drying, the compound was ground in a mixture of $CH_2Cl_2$ and diethyl ether, and filtered. 29.86 g (83% yield) of compound VII were obtained : m.p. 208-211°C (dec.) ; $R_{fA}$ 0.16.

Step 8 HCl.H-Gln-Trp-Ala-Val-Gly-OBzl (VIII)

29.71 g of Boc-Gln-Trp-Ala-Val-Gly-OBzl (VII) (39.62 mmol) were deblocked as described in this example, step 6. The crude product was suspended in a mixture of 200 ml of methanol, 100 ml of $CH_2Cl_2$ and 1000 ml of petroleum ether under mechanical stirring for 3 hours.

After filtration and washings with ethyl ether, 25.30 g (93% yield) of partially purified compound VIII were obtained : m.p. 205-207°C (modification at 162°C) ; $R_{fC}$ 0.44.

Step 9 Boc-Thr-Gln-Trp-Ala-Val-Gly-OBzl (IX)

8.04 g of Boc-Thr-OH (36.65 mmol) and 25.15 g of HCl. H-Gln-Trp-Ala-Val-Gly-OBzl (VIII) (36.65 mmol) were condensed as described in this example, step 1.

The crude product was then dissolved in DMF and poured dropwise in a 10% solution of citric acid at 0°C under mechanical stirring. After filtration and washings, the product was dried and then treated with a mixture of 300 ml methanol, 700 ml $CH_2Cl_2$ and 400 ml of petroleum ether for 2 hours under stirring.

After filtration, 24.47 g (78% yield) of compound IX were obtained : m.p. 216-220°C ; $R_{fB}$ 0.38.

Step 10 Boc-Thr-Gln-Trp-Ala-Val-Gly-OH (X)

21.47 g of Boc-Thr-Gln-Trp-Ala-Val-Gly-OBzl (IX) (25.23 mmol), dissolved in 1 : 1 mixture of anhydrous DMF and methanol, were hydrogenated in the presence of 5.36 g of 10% palladium-on-charcoal at 37°C under mechanical stirring, at atmospheric pressure.

After 90 minutes, the catalyst was removed by filtration and the solution concentrated in vacuo.

The product was suspended for 2 hours in a mixture of 400 ml of methanol, 1000 ml of $CH_2Cl_2$ and 1000 ml of petroleum ether.

After filtration the product was purified by CCD in the solvent system : water (20 l + 0.2 l NMM brought to pH 5 with AcOH)/ethyl acetate (22.5 l)/n-butanol (2.50 l)/DMF (1.5 l). Fractions containing pure product were collected, concentrated in vacuo and evaporated again from n-butanol.

The residue was ground in a mixture of ethanol, ethyl acetate and diethyl ether, giving 14.04 g (75% yield) of compound X : m.p. 208-210°C (dec.) ; $R_{fC}$ 0.70 ; $R_{fD}$ 0.69.

Compound X has also been obtained from compound VI by the following alternative route :

Step 7a Z-Gln-Trp-Ala-Val-Gly-OBzl (XI)

8.84 g of Z-Gln-OH (31.54 mmol) and 17.60 g of HCl. H-Trp-Ala-Val-Gly-OBzl (VI) (31.54 mmol) were condensed as described in this example, step 1.

The reaction mixture was filtered and the precipitate washed several times with DMF.

The filtrate and the washings were collected and evaporated in vacuo to small volume, then poured into a chilled solution of 10% citric acid.

The product was filtered, washed with water to neutrality, then dried under vacuum.

By precipitation from methanol/diethyl ether, 21.95 g (81% yield) of partially purified compound XI were obtained : m.p. 244-249°C ; $R_{fB}$ 0.38.

Step 10a Boc-Thr-Gln-Trp-Ala-Val-Gly-OH (X)

18 g of Z-Gln-Trp-Ala-Val-Gly-OBzl (XI) (22.96 mmol) and 9.94 g of Boc-Thr-OSu (H.T. Strey et al. (1972) J. Am. Chem. Soc. 94, 6170-6178) (33.1 mmol) dissolved in 450 ml of DMF were made to react at 40°C under hydrogen stream in the presence of 4.5 g of 10% palladium-on-charcoal.

After 3 hours the catalyst was removed by filtration and the solution was concentrated in vacuo.

The product was then purified as described in this example, step 10. 14.67 g (84% yield) of compound X were obtained.

Step 11 Boc-Leu-Met-NH$_2$ (XII)

64.82 g of Boc-Leu-OH · H$_2$O (260 mmol) were dissolved in 45 ml of anhydrous DMF and the solvent evaporated.

The oily Boc-Leu-OH so obtained was condensed with H-Met-NH$_2$ · HCl as described in this example, step 1.

After 3 hours the solution was filtered and the solvent removed in vacuo.

The crude oil, dissolved in 750 ml methanol, was added dropwise to 1.5 l of a 2.5% chilled solution of sodium bicarbonate.

The precipitate, filtered and washed with water to neutrality, was redissolved in 1 l of methanol, treated with activated carbon and filtered.

The solution was concentrated and the precipitate was filtered and washed with a cold mixture of ethyl acetate/ligroin 4/1. 83.58 g (92.8% yield) of compound XII were obtained : m.p. 158-159°C ; R$_{fA}$ 0.70.

Step 12 HCl.H-leu-Met-NH$_2$ (XIII)

80.79 g of Boc-Leu-Met-NH$_2$ (XII) (223 mmol) were deblocked as described in this example, step 2.

After 30 minutes, the solvent was evaporated in vacuo and the oily residue was ground in isopropanol. 66.56 g (theoretic yield) of solid compound XIII were obtained : m.p. 190-195°C ; R$_{fC}$ 0.33.

Step 13 Boc-Thr-Gln-Trp-Ala-Val-Gly-Leu-Met-NH$_2$ (XVIII)

To a solution containing 500 mg of Boc-Thr-Gln-Trp-Ala-Val-Gly-OH (X) (0.66 mmol) in 12 ml of anhydrous DMF, 0.074 ml (0.66 mmol) of NMM and 0.067 ml (0.66 mmol) of EEC were successively added at a temperature of −15°C. After stirring at this temperature for 2 minutes, a cold solution of 196 mg of HCl.H Leu-Met-NH$_2$ (XIII) (0.66 mmol) and 0.074 ml (0.66 mmol) of NMM in 11 ml of anhydrous DMF was added.

The reaction mixture was stirred for 45 minutes at −12°C and 60 minutes at 0°C, then was filtered from salts and evaporated.

The product, after evaporation of the solvent in vacuo, was precipitated by the addition of water.

The precipitate was filtered, washed with diethyl ether, then dissolved in DMF and poured dropwise into a 5% cold solution of sodium bicarbonate.

After filtration and washings with water to neutraliy, the product was redissolved in DMF and the solution concentrated to an oily residue.

After grinding in a mixture of methanol and diethyl ether, 440 mg (66% yield) of compound XVIII were obtained m.p. 188-200°C (foam) ; R$_{fC}$ 0.66 ; RT 15.90.

## EXAMPLE 2

Preparation of HCl. H-Thr-Gln-Trp-Ala-Val-Gly-Leu-Met-NH$_2$ (XIX)

260 mg of Boc-Thr-Gln-Trp-Val-Gly-Leu-Met-NH$_2$ (XVIII) (0.26 mmol) were deblocked as described in example 1, step 6.

The solvent was removed in vacuo and the residue was successively evaporated in vacuo 3 times from DMF.

The product was then isolated from a mixture of methanol and diethyl-ether.

The partially purified product was dissolved in DMF and the solution was diluted with water, filtered and concentrated in vacuo.

The residue was crushed with diethyl ether, giving 140 mg (58% yield) of compound XIX : m.p. 240°C (dec.) ; R$_{fD}$ 0.69 ; E$_{1.2}$ 0.61 ; RT 11.32.

In an analogous manner the following peptides have also been synthesized (the trifluoracetates were obtained by Boc-deblocking with CF$_3$COOH) ;

XX   H-Thr-Gln-Trp-Ala-Val-Gly-His(Dnp)-D-Leu-Met-NH$_2$ · HCl m.p. 190-200°C (dec) ; R$_{fC}$ 0.37 ; R$_{fD}$ 0.74 ; E$_{1.2}$ 0.70 ; RT 14.60.

XXI   H-Thr-Gln-Trp-Ala-Val-Gly-His-D-Leu-Met-NH$_2$ · CF$_3$COOH m.p. 168-170°C (foam) ; R$_{fC}$ 0.29 ;

$R_{fD}$ 0.65 ; $E_{1.2}$ 0.90 ; RT 10.38.

XXII   H-Thr-Gln-Trp-Ala-Val-Gly-His(Dnp)-Leu-Met-NH$_2$ · HCl $R_{fC}$ 0.37 ; $E_{1.2}$ 0.67 ; RT 14.42.

XXIII   H-Thr-Gln-Trp-Ala-Val-Gly-Phe-Leu-Met-NH$_2$ · CF$_3$COOH $R_{fC}$ 0.40 ; $R_{fD}$ 0.73 ; $E_{1.2}$ 0.36 ; RT 13.95.

XXIV   H-Thr-Gin-Trp-Ala-Val-Gly-Ser-Leu-Met-NH$_2$ · CF$_3$COOH $R_{fC}$ 0.34 ; $R_{fD}$ 0.65 ; $E_{1.2}$ 0.51 ; RT 10.91.

XXV   H-Lys-Thr-Gln-Trp-Ala-Val-Gly-Ala-Leu-Met-NH$_2$ · 2HCl. $R_{fD}$ 0.51 ; $E_{1.2}$ 0.71 ; RT 10.87.

XXVI   H-Arg-Thr-Gln-Trp-Ala-Val-Gly-Ala-Leu-Met-NH$_2$ · 2CF$_3$COOH m.p. 175-180°C (dec) ; $R_{fC}$, 0.28 ; $R_{fD}$ 0.47 ; $E_{1.2}$ 0.77 ; RT 11.04.

XXVII   H-Thr-Gln-Trp-Ala-Val-Gly-His(Dnp)-Leu-NH(CH$_2$)$_4$CH$_3$ · CF$_3$COOH m.p. ; $R_{fC}$ 0.42 ; $R_{fD}$ 0.78 ; $E_{1.2}$ 0.72 ; RT 17.64.

XXVIII   H-Thr-Gln-Trp-Ala-Val-Gly-His-Leu-NH(CH$_2$)$_4$CH$_3$ · 2CF$_3$COOH m.p. 200-211°C (dec) ; $R_{fC}$ 0.33 ; $R_{fD}$ 0.69 ; $E_{1.2}$ 0.66 ; RT 13.66.

XXXIX   H-Lys-Thr-Gln-Trp-Ala-Val-Gly-D-Ala-Leu-Met-NH$_2$ · CF$_3$COOH $R_{fC}$ 0.25 ; RT 12.26.

XXX   H-Thr-Gln-Trp-Ala-Val-Gly-His (Dnp)-Leu-NH$_2$ · CF$_3$COOH $R_{fC}$ 0.23 ; RT 14.11.

XXXI   H-Thr-Gln-Trp-Ala-Val-Gly-His-Leu-NH$_2$ · 2CF$_3$COOH $R_{fC}$ 0.1 ; RT 9.18.

XXXII   Boc-Thr-Gln-Trp-Ala-Val-Gly-His(Dnp)-Leu-NH$_2$ $R_{fC}$ 0.72 ; RT 17.2.

XXXIII   Boc-Thr-Gln-Trp-Ala-Val-Gly-His-Leu-NH$_2$ $R_{fC}$ 0.45 ; RT 14.0.

XXXIV   Boc-Thr-Gln-Trp-Ala-Val-Gly-D-Ala-Leu-Met-NH$_2$ $R_{fC}$ 0.78 ; RT 15.4.

XXXV   H-Thr-Gln-Trp-Ala-Val-Gly-D-Ser-Leu-Met-NH$_2$

XXXVI   H-Lys-Thr-Gln-Trp-Ala-Val-Gly-D-Ser-Leu-Met-NH$_2$

XXXVII   H-Thr-Gln-Trp-Ala-Val-Gly-D-Ala-Leu-Met-NH$_2$

XXXVIII   H-Lys-Thr-Gln-Trp-Ala-Val-Gly-Leu-Met-NH$_2$

XXXIX   H-Arg-Thr-Gln-Trp-Ala-Val-Gly-Leu-Met-NH$_2$

XL   H-Glp-Gln-Trp-Ala-Val-Gly-Leu-Met-NH$_2$

XLIII   H-Thr-Gln-Trp-Ala-Val-Gly-D-Leu-Met-NH$_2$

XLIV   H-Lys-Thr-Gln-Trp-Ala-Val-Gly-D-Leu-Met-NH$_2$

XLV   H-Lys-Asn-Gln-Trp-Ala-Val-Gly-Leu-NH(CH$_2$)$_4$CH$_3$

XLVI   H-Lys-Asn-Gln-Trp-Ala-Val-Gly-Leu-NHCH$_2$C$_6$H$_5$

IL   H-Thr-Gln-Trp-Ala-Val-D-Ala-His-Leu-NH$_2$

LI   H-Thr-Gln-Trp-Ala-Val-D-Ala-Leu-Met-NH$_2$

LII   H-Thr-Gln-Trp-Ala-Val-D-Ala-D-His-Leu-Met-NH$_2$

LIII   H-Thr-Gln-Trp-Ala-Val-Gly-D-His-Phe-Met-NH$_2$

LIV   H-Thr-Gln-Trp-Ala-Val-D-Ala-D-His-D-Leu-Met-NH$_2$

## Claims

1. A peptide of the general formula I A-B-Gln-Trp-Ala-Val-W-X-Y T wherein

A represents a hydrogen atom, a Boc group or a Lys or Arg residue,

B represents a valence bond or an Asn, Thr or Glp residue,

W represents a Gly or ala residue,

X represents a valence bond or a His ($R_1$), his ($R_1$), Phe, Ser, D-Ser, Ala or D-Ala residue.

Y represents a valence bond or a Leu, D-Leu, Phe or D-Phe residue,

T represents an NH$_2$, NH(CH$_2$)$_4$CH$_3$ or NHCH$_2$C$_6$H$_5$ group or a Met-$R_2$, Leu-$R_2$, Ile-$R_2$ or Nle-$R_2$ residue,

$R_1$ represents a hydrogen atom or a Tos, Dnp or Bzl group, and

$R_2$ represents an NH$_2$, OH, OMe or NH-NH$_2$ group.

with the provisos that

when A represents a Lys or Arg residue, then B does not represent a Glp residue,

when X represents a His residue, then Y-Z together do not represent Leu-Met-NH$_2$ or Phe-Met-NH$_2$ residues,

when X represents a His (Dnp) residue and A represents a βoc group, then β does not represent an Asn residue, and

when X represents a His (Dnp) residue, then β does not represent a Glp residue ; or a pharmaceutically acceptable salt of such a peptide.

2. Any one of the following peptides :

Boc-Thr-Gln-Trp-Ala-Val-Gly-Leu-Met-NH$_2$,

H-Thr-Gln-Trp-ala-Val-Gly-Leu-Met-NH$_2$,
H-Thr-Gln-Trp-Ala-Val-Gly-His(Dnp)-D-Leu-Met-NH$_2$,
H-Thr-Gln-Trp-Ala-Val-Gly-His-D-Leu-Met-NH$_2$,
H-Thr-Gln-Trp-Ala-Val-Gly-His(Dnp)-Leu-Met-NH$_2$,
H-Thr-Gln-Trp-Ala-Val-Gly-Phe-Leu-Met-NH$_2$,
H-Thr-Gln-Trp-Ala-Val-Gly-Ser-Leu-Met-NH$_2$,
H-Lys-Thr-Gln-Trp-Ala-Val-Gly-Ala-Leu-Met-NH$_2$,
H-Arg-Thr-Gln-Trp-Ala-Val-Gly-Ala-Leu-Met-NH$_2$,
H-Thr-Gln-Trp-Ala-Val-Gly-His(Dnp)-Leu-NH(CH$_2$)$_4$CH$_3$,
H-Thr-Gln-Trp-Ala-Val-Gly-His-Leu-NH(CH$_2$)$_4$CH$_3$,
H-Lys-Thr-Gln-Trp-Ala-Val-Gly-D-Ala-Leu-Met-NH$_2$,
H-Thr-Gln-Trp-Ala-Val-Gly-His(Dnp)-Leu-NH$_2$,
H-Thr-Gln-Trp-Ala-Val-Gly-His-Leu-NH$_2$,
Boc-Thr-Gln-Trp-Ala-Gly-His(Dnp)-Leu-NH$_2$
Boc-Thr-Gln-Trp-Ala-Val-Gly-His-Leu-NH$_2$
Boc-Thr-Gln-Trp-Ala-Val-Gly-D-Ala-Leu-Met-NH$_2$
H-Thr-Gln-Trp-Ala-Val-Gly-D-Ser-Leu-Met-NH$_2$,
H-Lys-Thr-Gln-Trp-Ala-Val-Gly-D-Ser-Leu-Met-NH$_2$,
H-Thr-Gln-Trp-Ala-Val-Gly-D-Ala-Leu-Met-NH$_2$,
H-Lys-Thr-Gln-Trp-Ala-Val-Gly-Leu-Met-NH$_2$,
H-Arg-Thr-Gln-Trp-Ala-Val-Gly-Leu-Met-NH$_2$,
H-Glp-Gln-Trp-Ala-Val-Gly-Leu-Met-NH$_2$,
H-Thr-Gln-Trp-Ala-Val-Gly-D-Leu-Met-NH$_2$,
H-Lys-Thr-Gln-Trp-Ala-Val-Gly-D-Met-NH$_2$,
H-Lys-Asn-Gln-Trp-Ala-Val-Gly-Leu-NH(CH$_2$)$_4$CH$_3$,
H-Lys-Asn-Gln-Trp-Ala-Val-Gly-Leu-NHCH$_2$C$_6$H$_5$,
H-Thr-Gln-Ala-Val-D-Ala-His-Leu-NH$_2$,
H-Thr-Gln-Trp-Ala-Val-D-Ala-Leu-Met-NH$_2$,
H-Thr-Gln-Trp-Ala-Val-D-Ala-D-His-Leu-Met-NH$_2$,
H-Thr-Gln-Trp-Ala-Val-Gly-D-His-Phe-Met-NH$_2$ and
H-Thr-Gln-Trp-Ala-Val-D-Ala-D-His-D-Leu-Met-NH$_2$ ;
or a pharmaceutically acceptable salt thereof.

3. A pharmaceutical composition comprising a peptide according to claim 1 or claim 2 or a pharmaceutically acceptable salt of such a peptide in admixture with a pharmaceutically acceptable diluent or carrier.

4. A process for the preparation of a peptide according to claim 1, the process comprising condensing amino acids and/or amino acid derivatives in the desired sequence and/or peptide segments containing these amino acids or their derivatives in the desired sequence to give the desired peptide, either an end carboxylic acid group or an end amino group being activated for the peptide linkage and the remaining groups being protected, and optionally deprotecting the resultant peptide and/or converting the resultant peptide into a pharmaceutically acceptable salt thereof.


**Patentansprüche**

1. Ein Peptid der allgemeinen Formel I A-B-Gln-Trp-Ala-Val-W-X-Y-T worin
A ein Wasserstoffatom, eine Boc-Gruppe oder einen Rest Lys oder Arg darstellt,
B eine Valenzbindung oder einen Rest Asn, Thr oder Glp darstellt,
W einen Rest Gly oder D-Ala darstellt,
X eine Valenzbindung oder einen Rest His (R$_1$), D-His (R$_1$), Phe, Ser, D-Ser, Ala oder D-Ala darstellt,
Y eine Valenzbindung oder einen Rest Leu, D-Leu, Phe oder D-Phe darstellt,
T eine Gruppe NH$_2$, NH(CH$_2$)$_4$CH$_3$ oder NHCH$_2$-C$_6$H$_5$ oder einen Rest Met-R$_2$, Leu-R$_2$, Ile-R$_2$ oder Nle-R$_2$ darstellt,
R$_1$ ein Wasserstoffatom oder eine Gruppe Tos, Dnp oder Bzl darstellt, und
R$_2$ eine Gruppe NH$_2$, OH, OMe oder NH-NH$_2$ darstellt, unter der Bedingung, dass wenn A einen Rest Lys oder Arg darstellt, B nicht einen Glp-Rest darstellt,
wenn X einen His-Rest darstellt, Y-Z gemeinsam nicht Leu-Met-NH$_2$- oder Phe-Met-NH$_2$-Reste darstellen,
wenn X einen His (Dnp)-Rest und A eine Boc-Gruppe darstellen, B nicht einen Asn-Rest darstellt und
wenn X einen His (Dnp)-Rest darstellt, B nicht einen Glp-Rest darstellt ;

oder ein pharmazeutisch annehmbares Salz eines solchen Peptids.

2. Jedes der nachfolgenden Peptide :

Boc-Thr-Gln-Trp-Ala-Val-Gly-Leu-Met-NH$_2$,

H-Thr-Gln-Trp-Ala-Val-Gly-Leu-met-NH$_2$,

H-Thr-Gln-Trp-Ala-Val-Gly-His(Dnp)-D-Leu-Met-NH$_2$,

H-Thr-Gln-Trp-Ala-Val-Gly-His-D-Leu-Met-Nh$_2$,

H-Thr-Gln-Trp-Ala-Val-Gly-His(Dnp)-Leu-Met-NH$_2$,

H-Thr-Gln-Trp-Ala-Val-Gly-Phe-Leu-Met-NH$_2$,

H-Thr-Gln-Trp-Ala-Val-Gly-Ser-Leu-Met-NH$_2$,

H-Lys-Thr-Gln-Trp-Ala-Val-Gly-Ala-Leu-Met-NH$_2$,

H-Arg-Thr-Gln-Trp-Ala-Val-Gly-Ala-Leu-Met-NH$_2$,

H-Thr-Gln-Trp-Ala-Val-Gly-His(Dnp)-Leu-NH(CH$_2$)$_4$CH$_3$,

H-Thr-Gln-Trp-Ala-Val-Gly-His-Leu-NH(CH$_2$)$_4$CH$_3$,

H-Lys-Thr-Gln-Trp-Ala-Val-Gly-D-Ala-Leu-Met-NH$_2$,

H-Thr-Gln-Trp-Ala-Val-Gly-His(Dnp)-Leu-NH$_2$,

H-Thr-Gln-Trp-Ala-Val-Gly-His-Leu-NH$_2$,

Boc-Thr-Gln-Trp-Ala-Gly-His(Dnp)-Leu-NH$_2$

Boc-Thr-Gln-Trp-Ala-Val-Gly-His-Leu-NH$_2$

Boc-Thr-Gln-Trp-Ala-Val-Gly-D-Ala-Leu-Met-NH$_2$

H-Thr-Gln-Trp-Ala-Val-Gly-D-Ser-Leu-Met-NH$_2$,

H-Lys-Thr-Gln-Trp-Ala-Val-Gly-D-Ser-Leu-Met-NH$_2$,

H-Thr-Gln-Trp-Ala-Val-Gly-D-Ala-Leu-Met-NH$_2$,

H-Lys-Thr-Gln-Trp-Ala-Val-Gly-Leu-Met-NH$_2$,

H-Arg-Thr-Gln-Trp-Ala-Val-Gly-Leu-Met-NH$_2$,

H-Glp-Gln-Trp-Ala-Val-Gly-Leu-Met-NH$_2$,

H-Thr-Gln-Trp-Ala-Val-Gly-D-Leu-Met-NH$_2$,

H-Lys-Thr-Gln-Trp-Ala-Val-Gly-D-Leu-Met-NH$_2$,

H-Lys-Asn-Gln-Trp-Ala-Val-Gly-Leu-NH(CH$_2$)$_4$CH$_3$,

H-Lys-Asn-Gln-Trp-Ala-Val-Gly-Leu-NHCH$_2$C$_6$H$_5$,

H-Thr-Gln-Trp-Ala-Val-D-Ala-His-Leu-NH$_2$,

H-Thr-Gln-Trp-Ala-Val-D-Ala-Leu-Met-NH$_2$,

H-Thr-Gln-Trp-Ala-Val-D-Ala-D-His-Leu-Met-NH$_2$,

H-Thr-Gln-Trp-Ala-Val-Gly-D-His-Phe-Met-NH$_2$ und

H-Thr-Gln-Trp-Ala-Val-D-Ala-D-His-D-Leu-Met-NH$_2$ ;

oder ein pharmazeutisch annehmbares Salz derselben.

3. Eine pharmazeutische Zusammensetzung, die ein Peptid gemäss Anspruch 1 oder Anspruch 2 oder ein pharmazeutisch annehmbares Salz eines solchen Peptids vermischt mit einem pharmazeutisch annehmbaren Verdünnungsmittel oder Träger umfasst.

4. Verfahren zur Herstellung eines Peptids gemäss Anspruch 1, welches Kondensieren von Aminosäuren und/oder Aminosäurederivaten in der gewünschten Reihenfolge und/oder von Peptidsegmenten, die diese Aminosäuren oder ihre Derivate in der gewünschten Reihenfolge enthalten, umfasst, wobei eine terminale Karboxylsäuregruppe oder eine terminale Aminogruppe für die Peptidbindung aktiviert wird und die restlichen Gruppen geschützt werden, und, falls gewünscht, die Schutzgruppe des sich ergebenden Peptids entfernt wird und/oder das sich ergebende Peptid in ein pharmazeutisch annehmbares Salz desselben umgesetzt wird.

**Revendications**

1. Un peptide de la formule générale I A-B-Gln-Trp-Ala-Val-W-X-Y-T où

A représente un atome d'hydrogène, un groupe Boc ou un reste Lys ou Arg,

B représente une liaison de valence ou un reste Asn, Thr ou Glp,

W représente un reste Gly ou D-Ala

X représente une liaison de valence ou un reste His (R$_1$), D-His (R$_1$), Phe, Ser, D-Ser, Ala ou D-Ala,

Y représente une liaison de valence ou un reste Leu, D-Leu, Phe ou D-Phe,

T représente un groupe NH$_2$, NH(CH$_2$)$_4$CH$_3$ ou NHCH$_2$-C$_6$H$_5$ ou un reste Met-R$_2$, Leu-R$_2$, Ile-R$_2$ ou Nle-R$_2$,

R$_1$ représente un atome d'hydrogène ou un groupe Tos, Dnp ou Bzl, et

R$_2$ représente un groupe NH$_2$, OH, OMe ou NH-NH$_2$, à condition que

si A représente un reste Lys ou Arg, B ne représente pas un reste Glp,

EP 0 339 193 B1

si X représente un reste His, Y-Z pris ensemble ne représentent pas des restes Leu-Met-NH$_2$ ou Phe-Met-NH$_2$,

si X représente un reste His (Dnp) et A représente un groupe Boc, B ne représente pas un reste Asn, et

si X représente un reste His (Dnp), B ne représente pas un reste Glp ; ou un sel de ce peptide pharmaceutiquement acceptable.

2. Chacun des peptides suivants :

Boc-Thr-Gln-Trp-Ala-Val-Gly-Leu-Met-NH$_2$,

H-Thr-Gln-Trp-Ala-Val-Gly-Leu-Met-NH$_2$,

H-Thr-Gln-Trp-Ala-Val-Gly-His(Dnp)-D-Leu-Met-NH$_2$,

H-Thr-Gln-Trp-Ala-Val-Gly-His-D-Leu-Met-NH$_2$,

H-Thr-Gln-Trp-Ala-Val-Gly-His(Dnp)-Leu-Met-NH$_2$,

H-Thr-Gln-Trp-Ala-Val-Gly-Phe-Leu-Met-NH$_2$,

H-Thr-Gln-Trp-Ala-Val-Gly-Ser-Leu-Met-NH$_2$,

H-Lys-Thr-Gln-Trp-Ala-Val-Gly-Ala-Leu-Met-NH$_2$,

H-Arg-Thr-Gln-Trp-Ala-Val-Gly-Ala-Leu-Met-NH$_2$,

H-Thr-Gln-Trp-Ala-Val-Gly-His(Dnp)-Leu-NH(CH$_2$)$_4$CH$_3$,

H-Thr-Gln-Trp-Ala-Val-Gly-His-Leu-NH(CH$_2$)$_4$CH$_3$,

H-Lys-Thr-Gln-Trp-Ala-Val-Gly-D-Ala-Leu-Met-NH$_2$

H-Thr-Gln-Trp-Ala-Val-Gly-His(Dnp)-Leu-NH$_2$,

H-Thr-Gln-Trp-Ala-Val-Gly-His-Leu-NH$_2$,

Boc-Thr-Gln-Trp-Ala-Gly-His(Dnp)-Leu-NH$_2$

Boc-Thr-Gln-Trp-Ala-Val-Gly-His-Leu-NH$_2$

Boc-Thr-Gln-Trp-Ala-Val-Gly-D-Ala-Leu-Met-NH$_2$

H-Thr-Gln-Trp-Ala-Val-Gly-D-Ser-Leu-Met-NH$_2$,

H-Lys-Thr-Gln-Trp-Ala-Val-Gly-D-Ser-Leu-Met-NH$_2$,

H-Thr-Gln-Trp-Ala-Val-Gly-D-Ala-Leu-Met-NH$_2$,

H-Lys-Thr-Gln-Trp-Ala-Val-Gly-Leu-Met-NH$_2$,

H-Arg-Thr-Gln-Trp-Ala-Val-Gly-Leu-Met-NH$_2$,

H-Glp-Gln-Trp-Ala-Val-Gly-Leu-Met-NH$_2$

H-Thr-Gln-Trp-Ala-Val-Gly-D-Leu-Met-NH$_2$,

H-Lys-Thr-Gln-Trp-Ala-Val-Gly-D-Leu-Met-NH$_2$,

H-Lys-Asn-Gln-Trp-Ala-Val-Gly-Leu-NH(CH$_2$)$_4$CH$_3$,

H-Lys-Asn-Gln-Trp-Ala-Val-Gly-Leu-NHCH$_2$C$_6$H$_5$,

H-Thr-Gln-6Trp-Ala-Val-D-Ala-His-Leu-NH$_2$,

H-Thr-Gln-Trp-Ala-Val-D-Ala-Leu-Met-NH$_2$,

H-Thr-Gln-Trp-Ala-Val-D-Ala-D-His-Leu-Met-NH$_2$,

H-Thr-Gln-Trp-Ala-Val-Gly-D-His-Phe-Met-NH$_2$ et

H-Thr-Gln-Trp-Ala-Val-D-Ala-D-His-D-Leu-Met-NH$_2$ ;

ou l'un de leurs sels pharmaceutiquement acceptables.

3. Une composition pharmaceutique comprenant un peptide selon la revendication 1 ou la revendication 2 ou un sel de ce peptide pharmaceutiquement acceptable mélangé avec un diluant ou un support pharmaceutiquement acceptable.

4. Procédé de fabrication d'un peptide selon la revendication 1, comprenant la condensation d'acides aminés et/ou de dérivés des acides aminés dans l'ordre désiré, et/ou de segments des peptides contenant ces acides aminés ou leurs dérivés dans l'ordre désiré pour obtenir le peptide désiré, étant activé un groupe terminal d'acide carboxylique ou un groupe aminique terminal pour la liaison du peptide et les groupes restants étant protégés, et, si désiré, en déprotégeant le peptide résultant et/ou en transformant le peptide résultant dans un sel de ce dernier pharmaceutiquement acceptable.

13